# EUROPEAN PATENT SPECIFICATION

(11) **EP 3 372 388 B1**
(45) Date of publication and mention of the grant of the patent: **22.04.2020**
(21) Application number: 16862348.6
(22) Date of filing: 28.10.2016
(51) Int. Cl.: B29C 67/00, B33Y 40/00, B33Y 50/02, C07C 225/06, C07D 251/30, C09D 4/00

(54) **METHOD FOR FORMING THREE-DIMENSIONAL OBJECT**
VERFAHREN ZUR FORMUNG EINES DREIDIMENSIONALEN OBJEKTS
PROCÉDÉ DE FABRICATION D'UN OBJET TRIDIMENSIONNEL

(30) Priority: 03.11.2015 KR 20150153963
(43) Date of publication of application: 12.09.2018
(73) Proprietor: LG Chem, Ltd., Yeongdeungpo-gu Seoul 07336 (KR)
(72) Inventor: KIM, Mi-Kyoung, Daejeon 34122 (KR); KIM, Joon-Hyung, Daejeon 34122 (KR); PARK, Sung-Eun, Daejeon 34122 (KR); BACK, Seung-A, Daejeon 34122 (KR)
(74) Representative: Goddar, Heinz J.
(86) International application number: PCT/KR2016/012286
(87) International publication number: WO 2017/078332

(56) References cited:
- EP-A1- 3 327 095
- WO-A1-2015/108768
- WO-A1-2015/111366
- KR-A- 20080 084 626
- KR-A- 20080 096 171
- KR-A- 20140 009 442
- US-A1- 2003 107 158
- US-A1- 2010 288 194
- US-B1- 9 138 981

## Description

### [Technical Field]

The present application claims the benefit of priority to Korean Patent Application No. 10-2015-0153963, filed on November 3, 2015.

The present invention according to claims 1 to 8 relates to a method for forming a three-dimensional object.

### [Background Art]

Today, in order to quickly respond to various needs of limitless competitive market in producing products, three-dimensional printing technique is quickly spreading. The reason is that companies solve technical problems during product developing process as soon as possible by selecting the three-dimensional technique, and therefore the cost can be reduced and concept design-to-complete product producing process can be minimized. The technique forming a three-dimensional object by an inkjet technique can accurately realize product shape through a very thin layer, and therefore it has an advantage that it can be very easily applied according to techniques and uses required from each industrial field.

When forming a three-dimensional object by the inkjet technique, a support ink should be used together with a structure body ink as a main ingredient. Accordingly, when making a structure in the form suspended in the air, the support ink temporarily forms a support at the lower part for supporting and later the support should be clearly removed.

EP 3 327 095, US 2003/107158, US 2010/288194 and US 9 138 981 refer for example to three-dimensional process wherein the support are based on different polymers and removed by dissolving or detaching.

### [Disclosure]

### [Technical Problem]

In the past, after forming a three-dimensional object, the support part should be removed by hands and the remained part should be removed by hands or a water jet one by one very inconveniently. Thus, there was a problem that productivity is reduced and it is uneconomical. Accordingly, a method for easily removing the support, not by hands, is required.

The present invention has been made keeping in mind the above problems in the related art, and an object of the present invention is to provide a method for forming a three-dimensional object, which can remove a support without damage to a structure body when the three-dimensional object is manufactured by an inkjet method such that the three-dimensional object can be simply and economically manufactured.

### [Technical Solution]

The present invention according to claims 1 to 8 provides a method for forming a three-dimensional object comprising the steps of:
a) forming a three-dimensional object comprising a structure body and a support by laminating a plurality of layers; and
b) removing the support by dissolving the support in a mixture solution of water and a polar organic solvent, wherein the support is manufactured through a step of curing an ink composition for a three-dimensional printing support comprising an amine-containing monomer and a curing agent, wherein the amine-containing monomer is the compound of the following Chemical Formula 1, 2, 3, 4, 5, 6 or 7.

### [Advantageous Effects]

According to the present invention, there is an advantage that when a three-dimensional object is manufactured by an inkjet method, a support can be removed without damage to a structure body such that the three-dimensional object can be simply and economically manufactured.

### [Mode for Invention]

The present invention relates to a method for forming a three-dimensional object. Hereinafter, the forming method will be described in detail for each step.

First, a step a) of forming a three-dimensional object comprising a structure body and a support by laminating a plurality of layers is performed.

The step a) is to form a three-dimensional object containing a structure body and a support by inkjet printing a structure body ink and a support ink for forming a three-dimensional object. When forming the three-dimensional object, the intensity of radiation can be controlled. For example, when forming a lower layer, the intensity of radiation is reduced to slow curing reaction thereby reducing curing shrinkage, and for upper layers, the intensity of radiation is increased for curing.

The step a) is to accurately print the three-dimensional object comprising a thin wall, an overhang and a working part by spraying a photo-curable resin in the form of a very thin layer. The structure body ink composition and the support ink composition are sprayed together for each layer, and the support ink composition allows forming shape such as an overhang, a cavity, a hole and the like. A printing head moves along the X and Y axes, and sprays the ink compositions on a molding plate. When the ink compositions for one layer are sprayed, the structure body ink composition and the support ink composition are immediately cured by a UV lamp on right and left of the head. For spraying to the next layer, the molding plate goes down and then the same procedure is repeated to form the final three-dimensional object.

In one embodiment of the present invention, the structure body may be manufactured by a step of curing a structure body ink composition, which comprises an acryl-based photo-curable resin, a curing agent, a polymerization inhibitor, and a photo-sensitizer; preferably, the acryl-based photo-curable resin may be at least one selected from the group consisting of dipentaerythritol hexaacrylate (DPHA), tripropylene glycol diacrylate (TPGDA), trimethylpropane triacrylate (TMPTA), isobornyl acrylate, benzyl acrylate, 1,6-hexanediol diacrylate, triethyleneglycol diacrylate, bisphenol A (EO)₄ diacrylate, glycerin (PO)₃ triacrylate, and pentaerythritol tetraacrylate, but not limited thereto.

In one embodiment of the present invention, the curing agent may vary depending on curing methods, and it may be any one used in the art without particular limitation. As a specific example of the curing agent, a photo-initiator may be used. The photo-initiator may be any one used in the art according to the used light source without particular limitation, but preferably, it may be Irgacure 819 (bisacrylphosphine-based), Darocur TPO (monoacrylphosphine-based), Irgacure 369 (α-aminoketone-based), Irgacure 184 (α-hydroxyketone-based), Irgacure 907 (α-aminoketone-based), Irgacure 2022 (bisacrylphosphine/α-hydroxyketone-based), Irgacure 2100 (phosphine oxide-based), or a commercial photo-initiator having a similar structure therewith, but not limited thereto.

In one embodiment of the present invention, the polymerization inhibitor may be at least one selected from the group consisting of a nitrosoamine-based polymerization inhibitor and a hydroquinone-based polymerization inhibitor; preferably, it may be at least one selected from the group consisting of monomethylether hydroquinone (MEHQ), N-nitrosophenyl hydroxamine, 2,5-bis(1,1,3,3-tetramethylbutyl)hydroquinone), 2,5-bis(1,1-dimethylbutyl)hydroquinone, nitrobenzene, butylated hydroxyl toluene, and diphenyl picryl hydrazyl (DPPH).

More preferably, it may be monomethylether hydroquinone (MEHQ), but not limited thereto.

In another embodiment of the present invention, the photo-sensitizer may be at least one selected from the group consisting of benzophenone and isopropyl thioxanthone (Darocur ITX), and preferably, it may be isopropyl thioxanthone (Darocur ITX), but not limited thereto.

In one embodiment of the present invention, more preferably, the structure body may be manufactured by the step of curing the structure body ink composition, which comprises dipentaerythritol hexaacrylate (DPHA), trimethylolpropane triacrylate (TMPTA), bisacrylphosphine-based curing agent (Irgacure 819), isopropyl thioxanthone (ITX), and monomethyl ether hydroquinone (MEHQ), but not limited thereto.

In another embodiment of the present invention, the support may be manufactured by a step of curing the ink composition for a three-dimensional printing support, which comprises an amine-containing monomer and a curing agent.

The amine-containing monomer may be any one used in the art without particular limitation, but preferably, it may be at least one compound of the following Chemical Formula 1 to 6.

First, as the amine-containing monomer of the present invention, the compound of the following Chemical Formula 1 may be used: 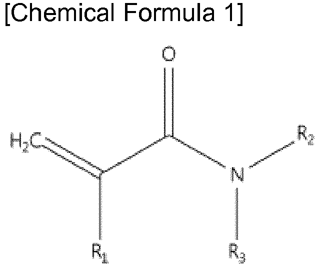 wherein, R₁ may be hydrogen or a methyl group, and R₂ and R₃ may be each independently hydrogen, a C₁-C₁₀ alkyl group, a vinyl group, an alkoxyl group, a cyclohexyl group, a phenyl group, a benzyl group, an alkylamine group, an alkyl ester group, or an alkyl ether group.

Further, preferably, in the Chemical Formula 1, R₂ and R₃ may be each independently hydrogen, methyl, ethyl, propyl, isopropyl, n-butyl, tert-butyl, 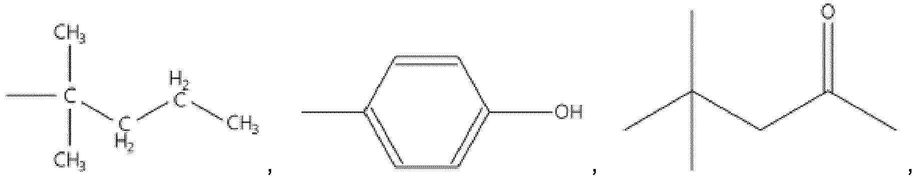 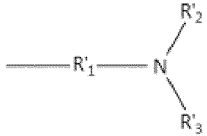 or -R"₁-O-R"₂;
R'₁ may be CH₂, CH₂CH₂, CH₂CH₂CH₂, CH(CH₃)CH₂, CH₂CH₂CH₂CH₂, CH₂C(CH₃)₂, or C(CH₃)₂CH₂CH₂, R'₂ and R'₃ may be each independently hydrogen, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃, or -CH=CH₂; and
R"₁ may be CH₂, CH₂CH₂, CH₂CH₂CH₂, CH(CH₂)CH₂, CH₂CH₂CH₂CH₂, CH₂C(CH₃)₂ or C(CH₃)₂CH₂CH₂, and R"₂ may be hydrogen, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃, or -CH=CH₂.

Further, preferably, the compound of the Chemical Formula 1 may be the compound of the following Chemical Formula 1a: 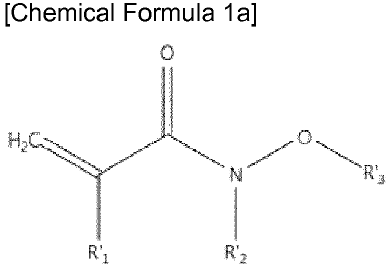 wherein, R'₁ may be hydrogen or a methyl group, and R'₂ and R'₃ may be each independently hydrogen, methyl, ethyl, propyl, isopropyl, n-butyl, tert-butyl, or -CH=CH₂.

Further, as the amine-containing monomer of the present invention, the compound of the Chemical Formula 2 may be used: 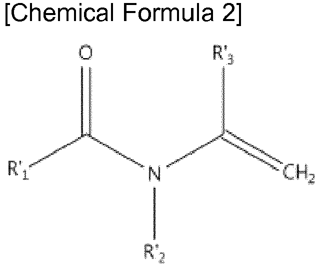 wherein, R'₁ and R'₂ may be each independently hydrogen, a C₁ to C₁₀ alkyl group, a vinyl group, an alkoxyl group, a cyclohexyl group, a phenyl group, a benzyl group, an alkylamine group, an alkyl ester group, or an alkyl ether group, and R'₃ may be hydrogen or a methyl group

Further, preferably, in the Chemical Formula 2, R'₁ and R'₂ may be each independently hydrogen, methyl, ethyl, propyl, isopropyl, n-butyl, tert-butyl, 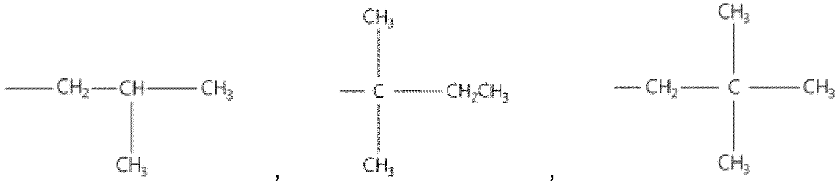 or

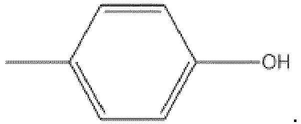

Further, as the amine-containing monomer of the present invention, the compound of the Chemical Formula 3 may be used: 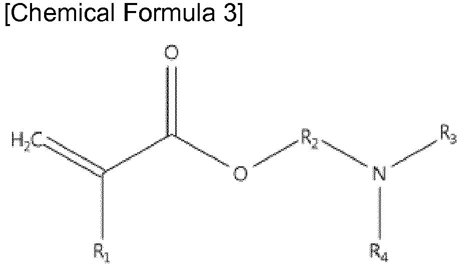 wherein, R₁ may be hydrogen or a methyl group, R₂ may be CH₂, CH₂CH₂, CH₂CH₂CH₂, CH(CH₂)CH₂, CH₂CH₂CH₂CH₂, CH₂C(CH₃)₂, or C(CH₃)₂CH₂CH₂, and R₃ and R₄ may be each independently hydrogen, methyl, ethyl, propyl, isopropyl, n-butyl, tert-butyl, -CH=CH₂, or -CH₂-CH=CH₂.

Further, as the amine-containing monomer of the present invention, the compound of the Chemical Formula 4 may be used: 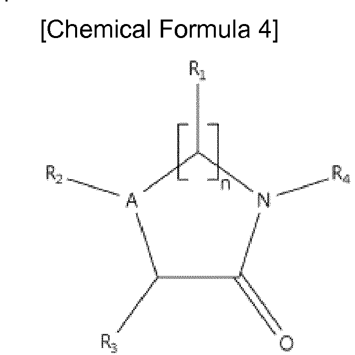 wherein, n may be an integer of 1 to 4, A is C, O, N or S, R₁, R₂ and R₃ may be each independently hydrogen or a C₁-C₁₀ alkyl group, and R₄ may be - CH=CH₂,

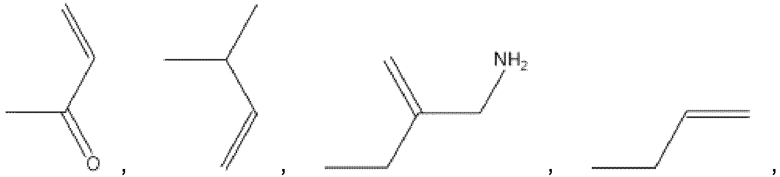

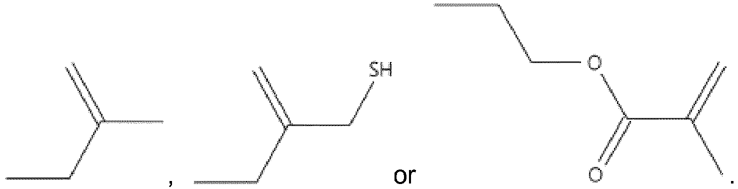

Further, preferably, the compound of the Chemical Formula 4 may be 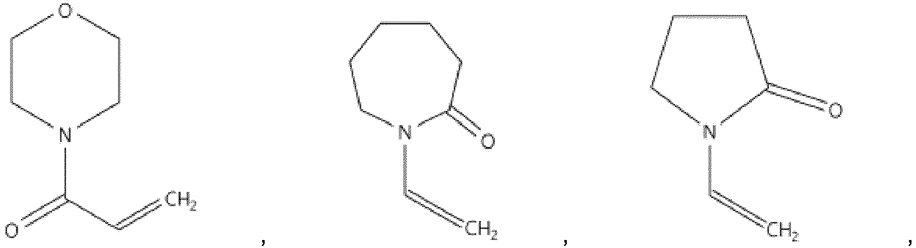 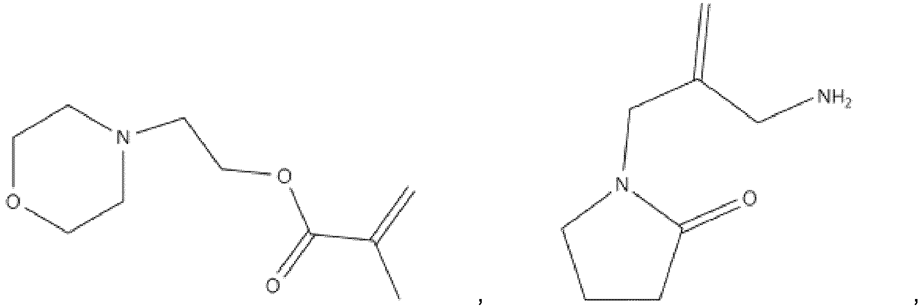 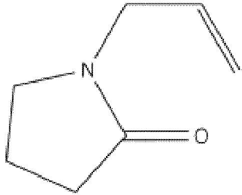 or

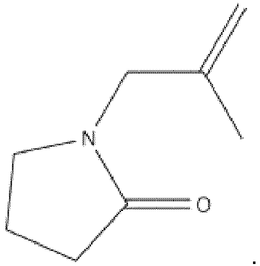

Further, as the amine-containing monomer of the present invention, it may be the compound of the following Chemical Formula 5, Chemical Formula 6 or Chemical Formula 7:

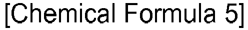

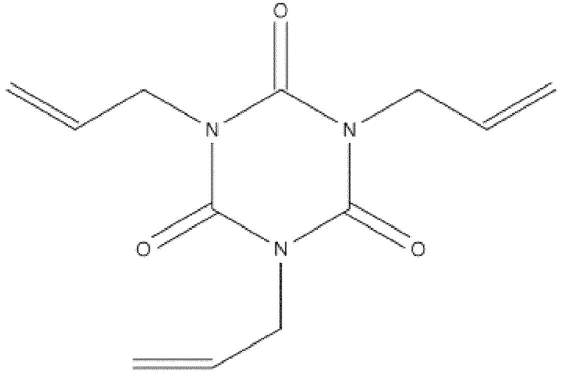

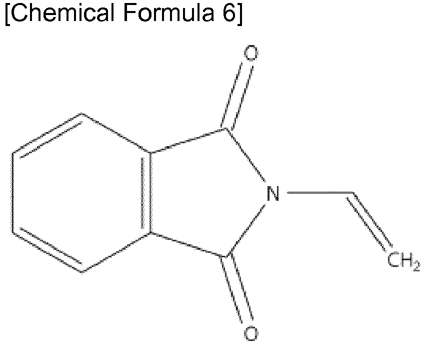

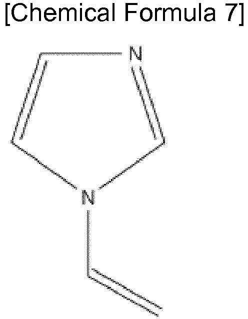

The amine-containing monomer may be contained in an amount of 10 to 99.9 wt% based on the total weight of the ink composition of the present invention. If the amount of the amine-containing monomer is less than 10 wt%, there may be a problem that the water solubility is not enough when removing a support, and if the amount is more than 99.9 wt%, there may be a problem that the curing property becomes worse.

The ink composition for a three-dimensional printing support may comprise a curing agent, and therefore, the composition may be used to a curing process by various curing methods.

In one embodiment of the present invention, the curing agent may vary depending on curing method, and it may be any one used in the art without particular limitation. As a specific example of the curing agent, a photo-initiator may be used. The photo-initiator may be any one used in the art according to the used light source without particular limitation, but preferably, it may be Irgacure 819 (bisacrylphosphine-based), Darocur TPO (monoacrylphosphine-based), Irgacure 369 (α-aminoketone-based), Irgacure 184 (α-hydroxyketone-based), Irgacure 907 (α-aminoketone-based), Irgacure 2022 (bisacrylphosphine/α-hydroxyketone-based), Irgacure 2100 (phosphine oxide-based), Darocur ITX (isopropyl thioxanthone) or a commercial photo-initiator having a similar structure therewith, but not limited thereto.

In one embodiment of the present invention, the curing agent may be contained in an amount of 0.01 to 20 wt% based on the total weight of the ink composition of the present invention, and preferably, it may be contained in an amount of 1 to 10 wt%. If the amount of the curing agent is less than 0.01 wt%, there may be a problem that curing is not happened, and if the amount is more than 20 wt%, there may be a problem that a head is blocked because the curing sensitivity is increased too much.

In another embodiment of the present invention, the ink composition for a three-dimensional printing support may further comprise a monomer comprising at least one of a vinyl group and an acryl group, and therefore, the composition may have a characteristic that it can control the sensitivity and properties such as the strength of a cured product (degree of softness or hardness).

In one embodiment of the present invention, the monomer comprising at least one of a vinyl group and an acryl group may be any one used in the art without particular limitation, but preferably, it may be at least one selected from the group consisting of vinyl acetate, 2-hydroxyethyl (meth)acrylate, 2-hydroxymethyl (meth)acrylate, 2-hydroxypropyl (meth)acrylate, 4-hydroxybutyl (meth)acrylate, ethyl-2-hydroxy acrylate, 2-(acryloyloxy)ethyl hydrogen succinate, and methacrylic acid.

In another embodiment of the present invention, the monomer comprising at least one of a vinyl group and an acryl group may be contained in an amount of 0.1 to 80 wt% based on the total weight of the ink composition of the present invention. If the amount of the monomer comprising at least one of a vinyl group and an acryl group is less than 0.1 wt%, there may be a problem that it is difficult to obtain enough effect according to addition of a monomer, and if the amount is more than 80 wt%, there may be a problem that the cured product is not dissolved in water.

In one embodiment of the present invention, the ink composition for a three-dimensional printing support may further comprise a water-soluble polymer, and therefore, the composition may have a characteristic that it controls the viscosity of an ink and makes the cured product be more easily dissolved in water.

In another embodiment of the present invention, the water-soluble polymer may be any one used in the art without particular limitation, but preferably, it may be at least one selected from the compounds of the following Chemical Formula 8a to Chemical Formula 8e: 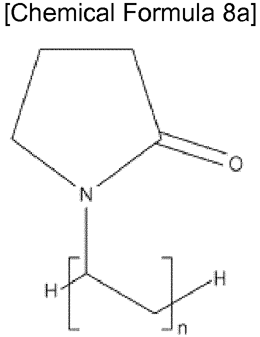 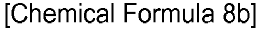 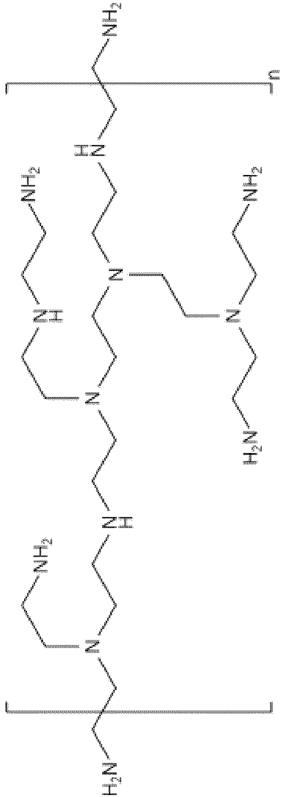 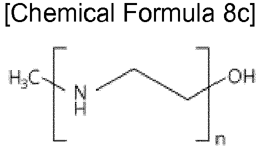 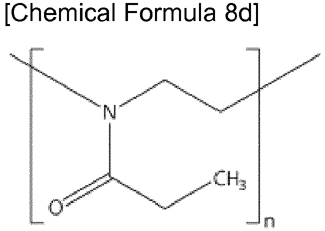 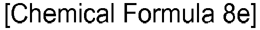 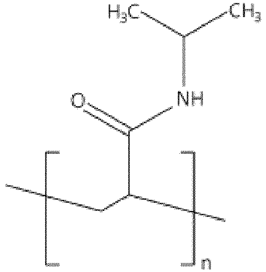 (wherein, n may be 50 to 25,000.)

In one embodiment of the present invention,
the water-soluble polymer may be contained in an amount of 0.1 to 30 wt% based on the total weight of the ink composition of the present invention. If the amount of the water-soluble polymer is less than 0.1 wt%, the effect of increasing the solubility according to the addition of the polymer may be poor, and if the amount is more than 30 wt%, there may be a problem that jetting is impossible because the viscosity of an ink becomes high.

In another embodiment of the present invention, the ink composition for a three-dimensional printing support may further comprise a vinyl ether compound, and therefore, when the composition is cured, shrinkage may be prevented.

In one embodiment of the present invention, the vinyl ether may preferably be 4-hydroxybutyl vinyl ether (HBVE), ethyl vinyl ether, butyl vinyl ether, cyclohexyl vinyl ether, 2-ethylhexyl vinyl ether, dodecyl vinyl ether, diethylene glycol divinyl ether, 1,4-cyclohexanedimethanol divinyl ether, triethyleneglycol divinyl ether, 1,4-butanediol divinyl ether and the like, but not limited thereto.

In another embodiment of the present invention, the vinyl ether compound may be contained in an amount of 0.1 to 50 wt% based on the total weight of the ink composition of the present invention. If the amount of the vinyl ether compound is less than 0.1 wt%, there may be a problem that the improvement over the shrinkage when curing is weak, and if the amount is more than 50 wt%, there may be a problem that the hardness and the strength of a membrane become weak and the curing sensitivity is reduced when curing.

In further another embodiment of the present invention, the ink composition for a three-dimensional printing support may further comprise additives in addition to the above composition. The additives may be a surfactant, a plasticizer, a polymerization inhibitor, an antifoaming agent, a diluent, a thermal stabilizer, a viscosity controlling agent and the like.

The additives may be contained in the minimum amount which can cause the above actions in terms of economics, and preferably, it may be contained in an amount of 0.1 to 5 wt% based on the whole ink composition.

In one embodiment of the present invention, preferably, the support may be manufactured by curing a support ink composition which comprises a (meth)acrylamide-based monomer, a vinyl-based monomer, a water-soluble polymer, and a curing agent; more preferably, it may be manufactured by curing a support ink composition which comprises dimethyl acrylamide, 1-vinyl-2-pyrrolidone (VP), polyvinyl pyrrolidone (PVP), or a bis acryl phosphine-based curing agent (Irgacure 819), but not limited thereto.

Then, a step b) of removing the support by dissolving the support in a mixture solution of water and a polar organic solvent is performed.

The step b) is to remove the support by dissolving the three-dimensional object comprising a structure body and a support, which is formed in the step a), in a mixture solution of water and a polar organic solvent.

In one embodiment of the present invention,
when performing the step b), the temperature of the mixture solution of water and a polar organic solvent may preferably be 20°C to 90°C, and more preferably 40°C to 60°C, but not limited thereto.

The step b) may be performed at room temperature, but it is preferred to increase the temperature of the solution within the above range for rapider removal.

In another embodiment of the present invention, when performing the step b), a step of stirring or sonicating water or the mixture solution of water and a polar organic solvent may be further performed to increase the removal rate of the support.

In one embodiment of the present invention, the polar organic solvent may be at least one selected from the group consisting of alcohols, glycols, glycol ethers, ketones, chlorines, N-methyl-2-pyrrolidone (NMP), dimethyl sulfoxide (DMSO), and acetonitrile, and it may preferably be at least one selected from the group consisting of methanol, ethanol, isopropyl alcohol, ethylene glycol, propylene glycol, ethylene glycol monobutyl ether, acetone, methyl ethyl ketone (MEK), chloroform, chlorobenzene, N-methyl-2-pyrrolidone (NMP), dimethyl sulfoxide (DMSO), and acetonitrile, but not limited thereto.

More preferably, it may be at least one selected from the group consisting of methanol, ethanol, isopropyl alcohol, ethylene glycol, propylene glycol, and ethylene glycol monobutyl ether, but not limited thereto.

In the mixture solution of water and a polar organic solvent, it may preferably be a mixture solution of water and alcohol-based solvent.

Then, a step c) of drying the support-removed three-dimensional object may be further performed, but not limited thereto.

In one embodiment of the present invention, dyring in the step c) may be performed by natural drying or drying means, and preferably, natural drying.

The drying means may be at least one selected from the group consisting of a heater, an oven, and a heat gun, but not limited thereto.

### [Best Mode Carrying out the Invention]

A better understanding of the present invention may be obtained via the following examples that are set forth to illustrate, but are not to be construed as limiting the present invention. The scope of the present invention is described in the appended claims, and includes all modifications within ranges and meanings equivalent to the claims. Unless otherwise stated, "%" and "part" showing the amounts in the following examples and comparative examples are based on mass

### Example

### Example 1 (not according to the invention)

### a) Step of forming three-dimensional object comprising structure body and support by laminating a plurality of layers

8 g of Dipentaerythritol hexaacrylate (DPHA), 68.5 g of trimethylolpropane triacrylate (TMPTA), 3.5 g of Irgacure 819, 1 g of isopropyl thioxanthone (ITX), and 0.5 g of monomethyl ether hydroquinone (MEHQ) were mixed to prepare a structure body ink composition.

Further, 49.2 g of dimethyl acrylamide, 32.8 g of 1-vinyl-2-pyrrolidone (VP), 14 g of polyvinyl pyrrolidone (PVP), and 4 g of Irgacure 819 were mixed to prepare a support ink composition.

The structure body ink composition and the support ink composition were subjected to three-dimensional printing by an inkjet method (Manufactured by Spectra, Apollo II, head : Dimatix 30 pL 128 nozzle head) to form a three-dimensional object, and curing was performed by using a 395 nm LED (2000 mJ/cm²).

### b) Step of removing support

The three-dimensional object formed in the step a) is dissolved in water of 50°C to remove a support.

### c) Step of drying support-removed three-dimensional object

The support-removed three-dimensional object was dried for 10 min at room temperature by blowing dry air.

### Example 2

The procedure of Example 1 was repeated except for using a mixed solution of water and ethanol (50:50) instead of water in the step b).

### Comparative Example 1

The procedure of Example 1 was repeated except for using ethanol instead of water in the step b).

### Comparative Example 2

The procedure of Example 1 was repeated except for using chlorobenzene instead of water in the step b).

### Comparative Example 3

The procedure of Example 1 was repeated except for using acetonitrile instead of water in the step b).

### Comparative Example 4

The procedure of Example 1 was repeated except for using acetone instead of water in the step b).

### Comparative Example 5

The procedure of Example 1 was repeated except for using ether instead of water in the step b).

### Comparative Example 6

The procedure of Example 1 was repeated except for using toluene instead of water in the step b).

Whether the support is removed or not and the structure body is damaged or not in Example 1 and 2 and Comparative Example 1 to 6 were checked, and the results are shown in the following Table 1.

**Table 1**

| | Solvent for removal | Whether support is removed or not (○, X) | Whether structure body is damaged or not (○, X) | Note |
|---|---|---|---|---|
| Example 1 | Water | ○ | X | |
| Example 2 | Water+Ethanol (50:50) | ○ | X | |
| Comparative Example 1 | Ethanol | ○ | Δ | |
| Comparative Example 2 | Chlorobenzene | ○ | ○ | |
| Comparative Example 3 | Acetonitrile | ○ | ○ | |
| Comparative Example 4 | Acetone | ○ | Δ | |
| Comparative Example 5 | Ether | X | X | Nonpolar solvent |
| Comparative Example 6 | Toluene | X | ○ | Nonpolar solvent |

*Whether support is removed or not:
When immersing the three-dimensional object comprising the support in the corresponding solvent, if the support is dissolved in the solvent, marked as O, and if it is not dissolved, marked as X.
** Whether structure body is removed or not:
If a part of the structure body is dissolved, marked as O, if the structure body is damaged, for example, bent or discolored, marked as Δ, and if the structure body is not damaged at all, marked as X.

In the cases of using only water and using the mixture solution of water and an organic solvent as a removal solvent like Examples 1 and 2, the support was removed without damage of the structure body. However, in the case of removing the support with only a polar organic solvent like Comparative Examples 1 to 4, the structure body was partially dissolved or damaged. In the case of using a nonpolar organic solvent like Comparative Examples 5 and 6, the support was not dissolved.

## Claims

1. A method for forming a three-dimensional object comprising the steps of:
a) forming a three-dimensional object comprising a structure body and a support by laminating a plurality of layers; and
b) removing the support by dissolving the support in a mixture solution of water and a polar organic solvent,
wherein the support is manufactured through a step of curing an ink composition for a three-dimensional printing support comprising an amine-containing monomer and a curing agent,
wherein the amine-containing monomer is
the compound of the following Chemical Formula 1: 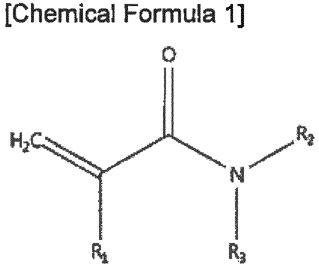 wherein, R₁ is hydrogen or a methyl group, and R₂ and R₃ are each independently hydrogen, a C₁-C₁₀ alkyl group, a vinyl group, an alkoxyl group, a cyclohexyl group, a phenyl group, a benzyl group, an alkylamine group, an alkyl ester group, or an alkyl ether group;
the compound of the following Chemical Formula 2: 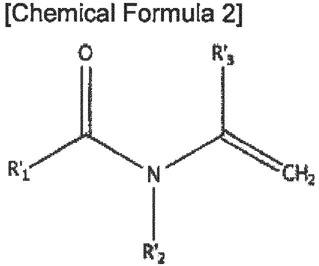 wherein, R'₁ and R'₂ are each independently hydrogen, a C₁ to C₁₀ alkyl group, a vinyl group, an alkoxyl group, a cyclohexyl group, a phenyl group, a benzyl group, an alkylamine group, an alkyl ester group or an alkyl ether group, and R'₃ is hydrogen or a methyl group;
the compound of the following Chemical Formula 3: 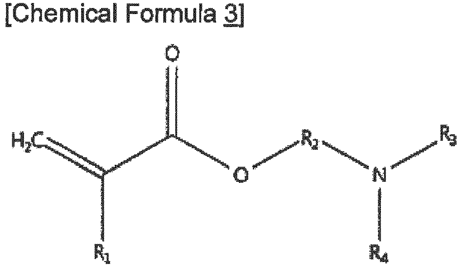 wherein, R₁ is hydrogen or a methyl group, R₂ is CH₂, CH₂CH₂, CH₂CH₂CH₂, CH(CH₂)CH₂, CH₂CH₂CH₂CH₂, CH₂C(CH₃)₂ or C(CH₃)₂CH₂CH₂, and R₃ and R₄ are each independently hydrogen, methyl, ethyl, propyl, isopropyl, n-butyl, tert-butyl, -CH=CH₂, or -CH₂-CH=CH₂;
the compound of the following Chemical Formula 4; 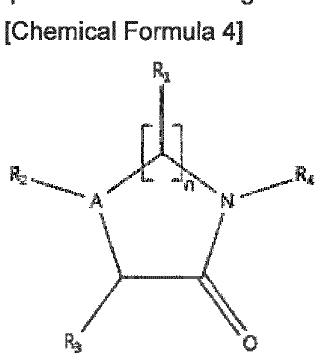 wherein, n is an integer of 1 to 4, A is C, O, N or S, R₁, R₂ and R₃ are each independently hydrogen or a C₁-C₁₀ alkyl group, and R₄ is -CH=CH₂, 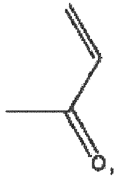 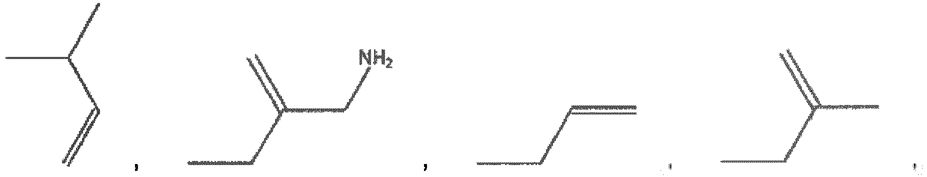 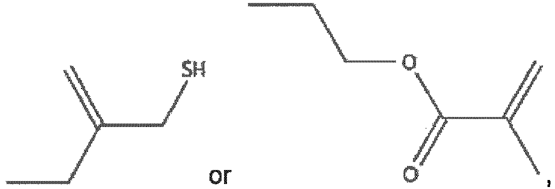 the compound of the following Chemical Formula 5; Chemical Formula 6; or Chemical Formula 7:

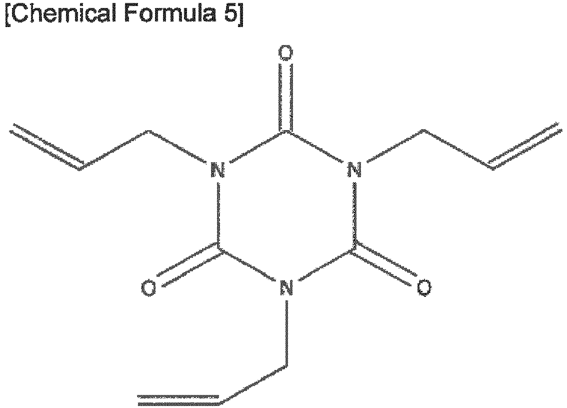

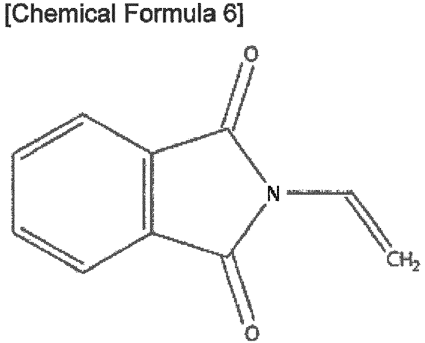

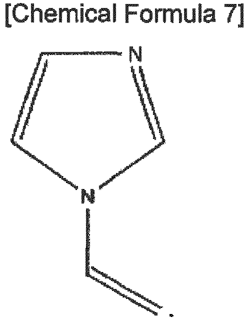

2. The method for forming a three-dimensional object according to claim 1, wherein the polar organic solvent is at least one selected from the group consisting of methanol, ethanol, isopropyl alcohol, ethylene glycol, propylene glycol, and ethylene glycol monobutyl ether.

3. The method for forming a three-dimensional object according to claim 1, wherein the ink composition for a three-dimensional printing support further comprises a monomer comprising at least one of a vinyl group and an acryl group.

4. The method for forming a three-dimensional object according to claim 1, wherein R₂ and R₃ in Chemical Formula 1 are each independently hydrogen, methyl, ethyl, propyl, isopropyl, n-butyl, tert-butyl, 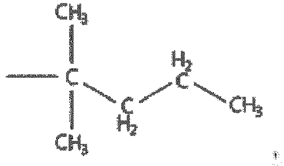 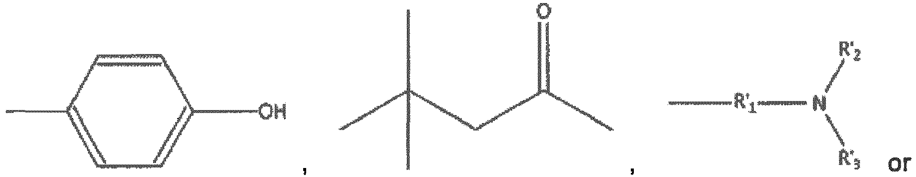 -R"₁-O-R"₂, wherein R'₁ is CH₂, CH₂CH₂, CH₂CH₂CH₂, CH(CH₂)CH₂, CH₂CH₂CH₂CH₂, CH₂C(CH₃)₂ or C(CH₃)₂CH₂CH₂, R'₂ and R'₃ are each independently hydrogen, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃ or -CH=CH₂, R"₁ is CH₂, CH₂CH₂, CH₂CH₂CH₂, CH(CH₂)CH₂, CH₂CH₂CH₂CH₂, CH₂C(CH₃)₂ or C(CH₃)₂CH₂CH₂, and R"₂ is hydrogen, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃, or -CH=CH₂.

5. The method for forming a three-dimensional object according to claim 1, wherein the compound of the Chemical Formula 1 is the compound of the following Chemical Formula 1a: 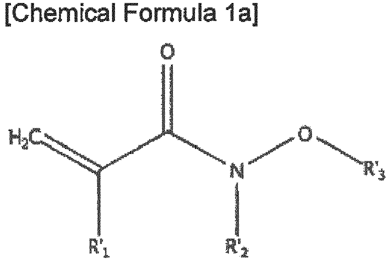 wherein, R'₁ is hydrogen or a methyl group, and R'₂ and R'₃ are each independently hydrogen, methyl, ethyl, propyl, isopropyl, n-butyl, tert-butyl, or - CH=CH₂.

6. The method for forming a three-dimensional object according to claim 1, wherein R'₁ and R'₂ in Chemical Formula 2 are each independently hydrogen, methyl, ethyl, propyl, isopropyl, n-butyl, tert-butyl,

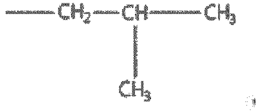

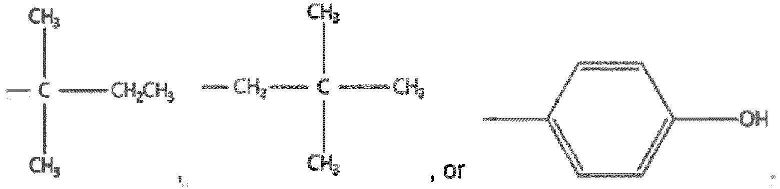

7. The method for forming a three-dimensional object according to claim 1, wherein the compound of the Chemical Formula 4 is a compound selected from the following compounds:

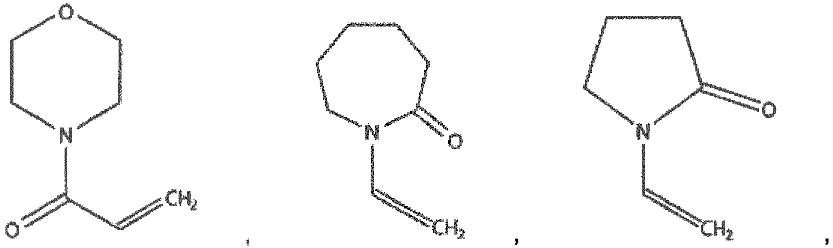

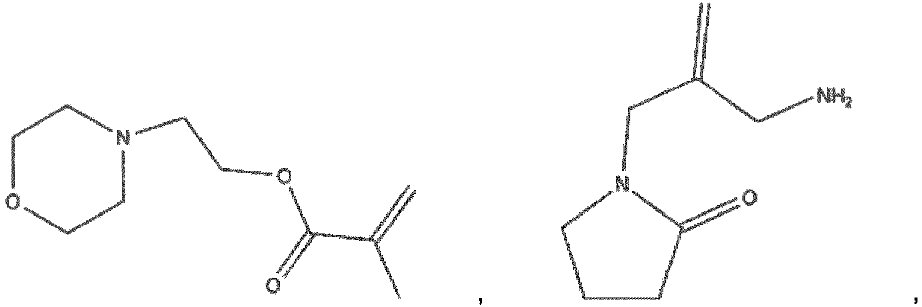

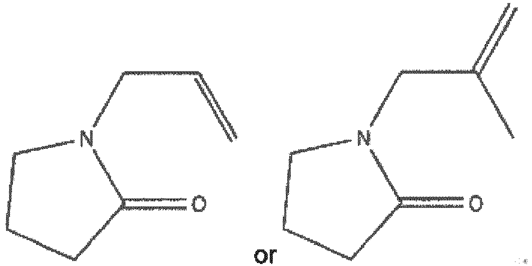

8. The method for forming a three-dimensional object according to claim 1, wherein the amine-containing monomer is contained in an amount of 10 to 99.9 wt% based on the total weight of the composition.

9. The method for forming a three-dimensional object according to claim 1, wherein the curing agent is contained in an amount of 0.01 to 20 wt% based on the total weight of the composition.

## Patentansprüche

1. Verfahren zum Bilden eines dreidimensionalen Objekts, umfassend die Schritte:
a) Bilden eines dreidimensionalen Objekts umfassend einen Strukturkörper und einen Träger durch Laminieren einer Vielzahl von Schichten; und
b) Entfernen des Trägers durch Auflösen des Trägers in einer Mischungslösung aus Wasser und einem polaren organischen Lösungsmittel,
wobei der Träger durch einen Schritt eines Härtens einer Tintenzusammensetzung für einen dreidimensionalen Druckträger, umfassend ein Amin-enthaltendes Monomer und ein Härtungsmittel, hergestellt wird,
wobei das Amin-enthaltende Monomer
die Verbindung der folgenden chemischen Formel 1 : 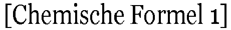 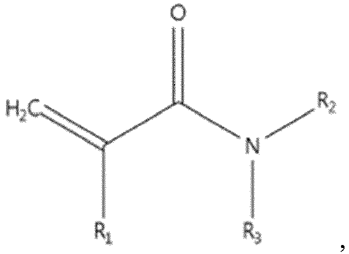 wobei R₁ Wasserstoff oder eine Methylgruppe ist und R₂ und R₃ jeweils unabhängig Wasserstoff, eine C₁-C₁₀-Alkylgruppe, eine Vinylgruppe, eine Alkoxylgruppe, eine Cyclohexylgruppe, eine Phenylgruppe, eine Benzylgruppe, eine Alkylamingruppe, eine Alkylestergruppe oder eine Alkylethergruppe sind;
die Verbindung der folgenden chemischen Formel 2: 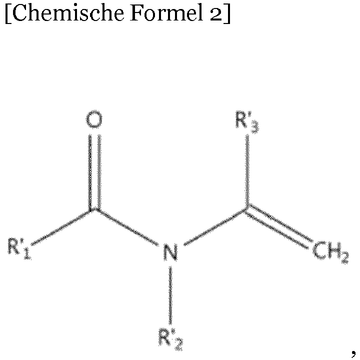 wobei R'₁ und R'₂ jeweils unabhängig Wasserstoff, eine C₁-C₁₀-Alkylgruppe, eine Vinylgruppe, eine Alkoxylgruppe, eine Cyclohexylgruppe, eine Phenylgruppe, eine Benzylgruppe, eine Alkylamingruppe, eine Alkylestergruppe oder eine Alkylethergruppe sind und R'₃ Wasserstoff oder eine Methylgruppe ist;
die Verbindung der folgenden chemischen Formel 3: 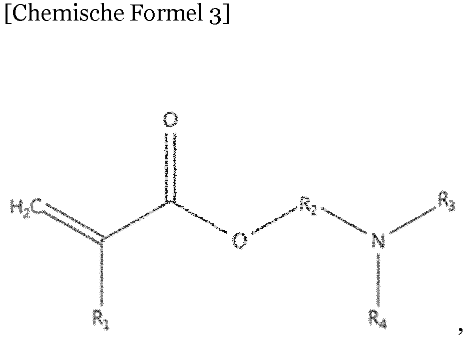 wobei R¹ Wasserstoff oder eine Methylgruppe ist, R₂ CH₂, CH₂CH₂, CH₂CH₂CH₂, CH(CH₂)CH₂, CH₂CH₂CH₂CH₂, CH₂C(CH₃)₂ oder C(CH₃)₂CH₂CH₂ ist und R₃ und R₄ jeweils unabhängig Wasserstoff, Methyl, Ethyl, Propyl, Isopropyl, n-Butyl, tert-Butyl, -CH=CH₂ oder -CH₂-CH=CH₂ ist;
die Verbindung der folgenden chemischen Formel 4: 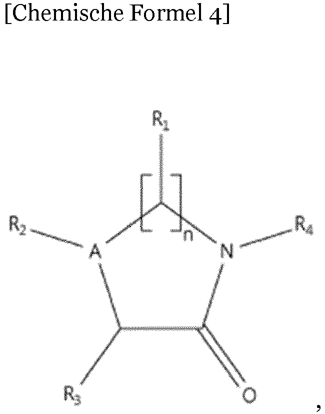 wobei n eine ganze Zahl von 1 bis 4 ist, A C, O, N oder S ist, R₁, R₂ und R₃ jeweils unabhängig Wasserstoff oder eine C₁-C₁₀-Alkylgruppe sind und R₄ -CH=CH₂, 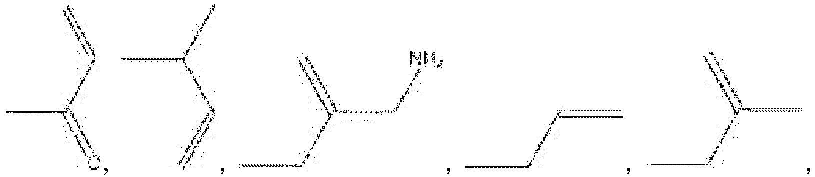 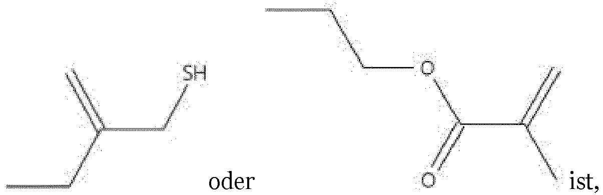 die Verbindung der folgenden chemischen Formel 5; chemischen Formel 6; oder chemischen Formel 7:

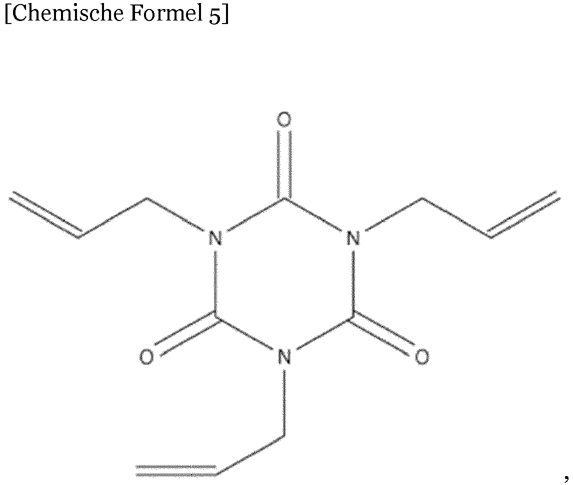

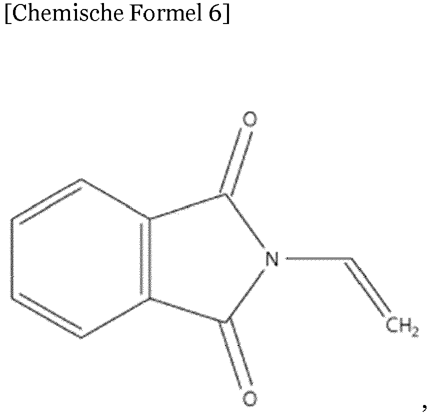

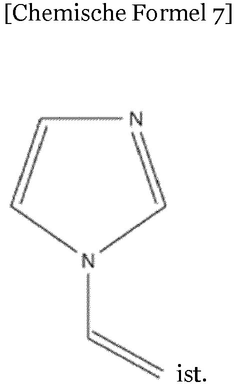

2. Verfahren zum Bilden eines dreidimensionalen Objekts nach Anspruch 1, wobei das polare organische Lösungsmittel wenigstens eines ist, ausgewählt aus der Gruppe bestehend aus Methanol, Ethanol, Isopropylalkohol, Ethylenglykol, Propylenglykol und Ethylenglykolmonobutylether.

3. Verfahren zum Bilden eines dreidimensionalen Objekts nach Anspruch 1, wobei die Tintenzusammensetzung für einen dreidimensionalen Druckträger ferner ein Monomer umfasst, das wenigstens eines einer Vinylgruppe und einer Acrylgruppe umfasst.

4. Verfahren zum Bilden eines dreidimensionalen Objekts nach Anspruch 1, wobei R₂ und R₃ in chemischer Formel 1 jeweils unabhängig Wasserstoff, Methyl, Ethyl, Propyl, Isopropyl, n-Butyl, tert-Butyl, 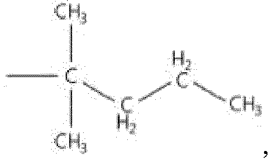 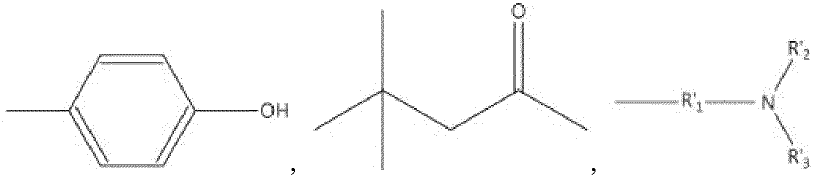 oder -R"₁-O-R"₂ ist, wobei R'₁ CH₂, CH₂CH₂, CH₂CH₂CH₂, CH(CH₂)CH₂, CH₂CH₂CH₂CH₂, CH₂C(CH₃)₂ oder C(CH₃)₂CH₂CH₂ ist, R'₂ und R'₃ jeweils unabhängig Wasserstoff, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃ oder -CH=CH₂ sind, R"₁ CH₂, CH₂CH₂, CH₂CH₂CH₂, CH(CH₂)CH₂, CH₂CH₂CH₂CH₂, CH₂C(CH₃)₂ oder C(CH₃)₂CH₂CH₂ ist, und R"₂ Wasserstoff, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃ oder -CH=CH₂ ist.

5. Verfahren zum Bilden eines dreidimensionalen Objekts nach Anspruch 1, wobei die Verbindung der chemischen Formel 1 die Verbindung der folgenden chemischen Formel 1a ist: 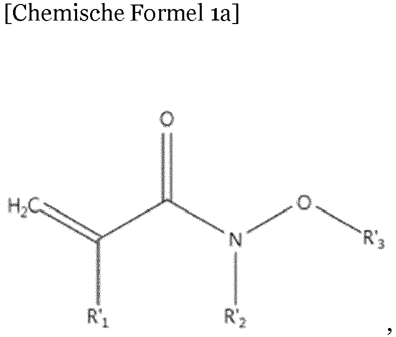 wobei R'₁ Wasserstoff oder eine Methylgruppe ist und R'₂ und R'₃ jeweils unabhängig Wasserstoff, Methyl, Ethyl, Propyl, Isopropyl, n-Butyl, tert-Butyl oder -CH=CH₂ sind.

6. Verfahren zum Bilden eines dreidimensionalen Objekts nach Anspruch 1, wobei R'₁ und R'₂ in chemischer Formel 2 jeweils unabhängig Wasserstoff, Methyl, Ethyl, Propyl, Isopropyl, n-Butyl, tert-Butyl, 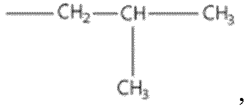 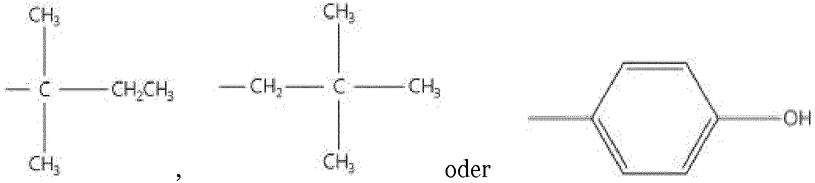 sind.

7. Verfahren zum Bilden eines dreidimensionalen Objekts nach Anspruch 1, wobei die Verbindung der chemischen Formel 4 eine Verbindung ausgewählt aus den folgenden Verbindungen ist: 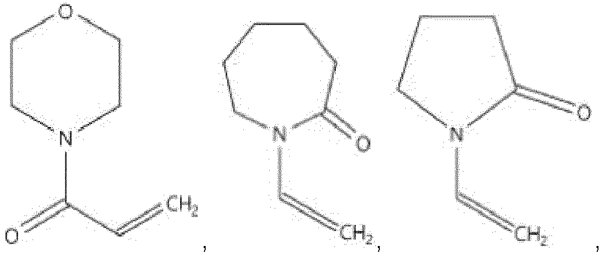 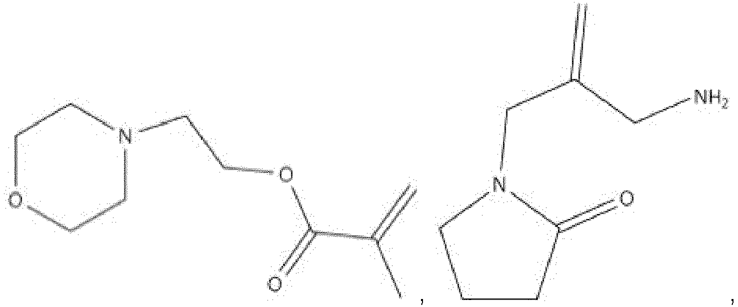 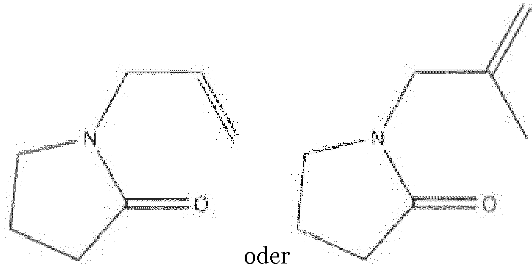 ist.

8. Verfahren zum Bilden eines dreidimensionalen Objekts nach Anspruch 1, wobei das Amin-enthaltende Monomer in einer Menge von 10 bis 99,9 Gew.-%, basierend auf dem Gesamtgewicht der Zusammensetzung, enthalten ist.

9. Verfahren zum Bilden eines dreidimensionalen Objekts nach Anspruch 1, wobei das Härtungsmittel in einer Menge von 0,01 bis 20 Gew.-%, basierend auf dem Gesamtgewicht der Zusammensetzung, enthalten ist.

## Revendications

1. Procédé de fabrication d'un objet tridimensionnel comprenant les étapes consistant à :
a) former un objet tridimensionnel comprenant un corps de structure et un support en stratifiant une pluralité de couches ; et
b) enlever le support en le dissolvant dans une solution de mélange d'eau et d'un solvant organique polaire,
dans lequel le support est fabriqué par une étape de durcissement d'une composition d'encre pour un support d'impression tridimensionnel comprenant un monomère contenant une amine et un agent de durcissement,
où le monomère contenant l'amine est le composé de la formule chimique 1 suivante : 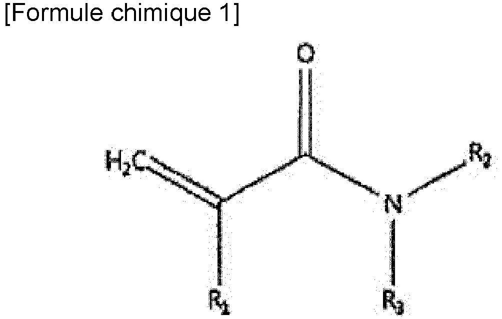 dans laquelle, R₁ représente l'hydrogène ou un groupe méthyle, et R₂ et R₃ représentent chacun indépendamment l'hydrogène, un groupe alkyle en C₁ à C₁₀, un groupe vinyle, un groupe alcoxyle, un groupe cyclohexyle, un groupe phényle, un groupe benzyle, un groupe alkylamine, un groupe ester d'alkyle, ou un groupe éther d'alkyle ;
le composé de la formule chimique 2 suivante : 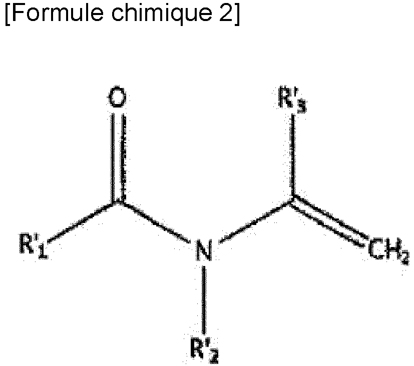 dans laquelle, R'₁ et R'₂ représentent chacun indépendamment l'hydrogène, un groupe alkyle en C₁ à C₁₀, un groupe vinyle, un groupe alcoxyle, un groupe cyclohexyle, un groupe phényle, un groupe benzyle, un groupe alkylamine, un groupe ester d'alkyle ou un groupe éther d'alkyle et R'₂ représente l'hydrogène ou un groupe méthyle ;
le composé de la formule chimique 3 suivante : 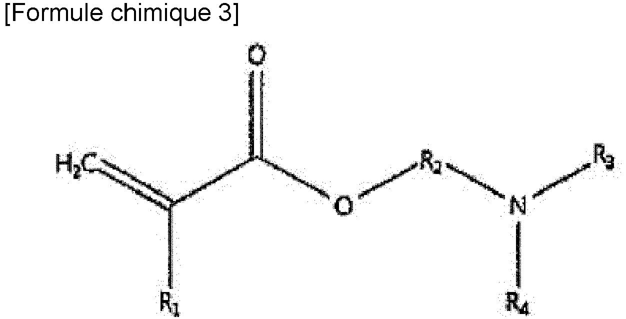 dans laquelle formule, R₁ représente l'hydrogène ou un groupe méthyle, R₂ représente CH₂, CH₂CH₂, CH₂CH₂CH₂, CH(CH₂)CH₂, CH₂CH₂CH₂CH₂, CH₂C(CH₃)₂ ou C(CH₃)₂CH₂CH₂ et R₃ et R₄ représentent chacun indépendamment l'hydrogène, le méthyle, l'éthyle, le propyle, l'isopropyle, le n-10-butyle, le tert-butyle, -CH = CH₂ ou -CH₂-CH = CH₂ ;
le composé de la formule chimique 4 suivante ; 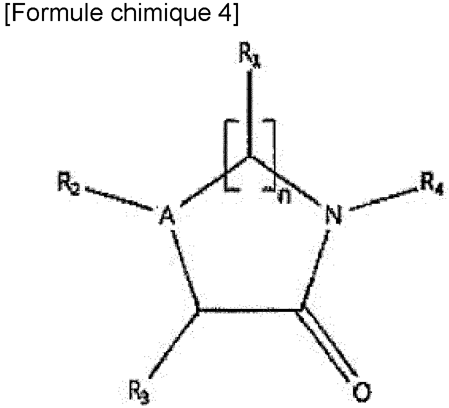 dans laquelle n représente un nombre entier valant de 1 à 4, A représente C, O, N. ou S, R₁ , R₂ et R₃ représentent chacun
indépendamment l'hydrogène ou un groupe alkyle en C₁ à C₁₀, et R₄ représente CH = CH₂, 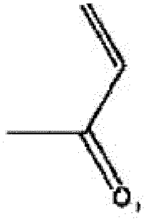 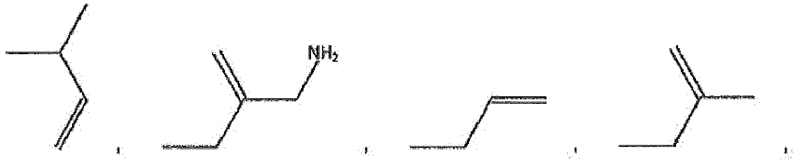 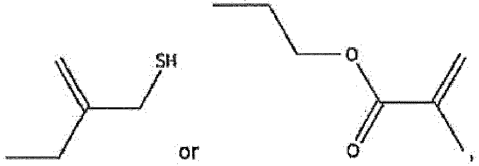 or = ou
le composé de la formule chimique 5; de la formule chimique 6 suivante ;
ou de la formule chimique 7 suivante ;

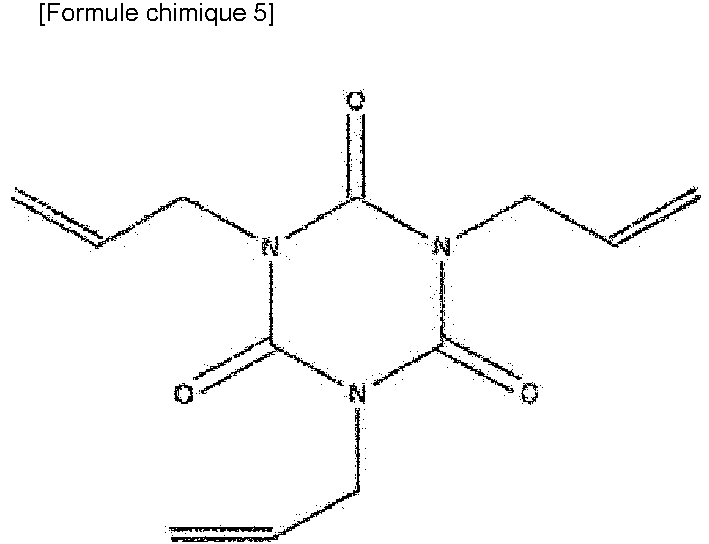

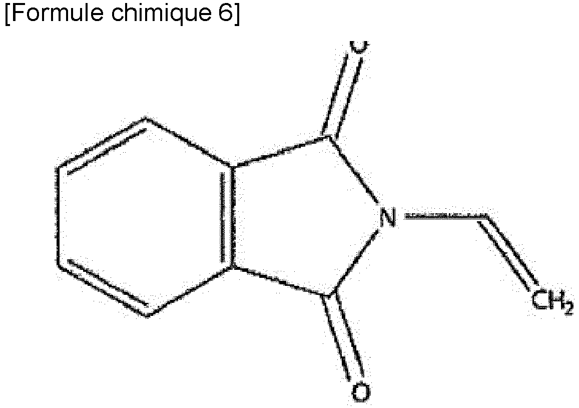

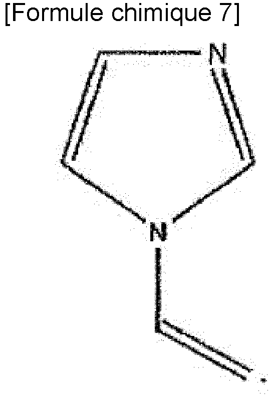

2. Procédé de fabrication d'un objet tridimensionnel selon la revendication 1, dans lequel le solvant organique polaire est au moins un solvant sélectionné dans le groupe constitué de méthanol, d'éthanol, d'alcool isopropylique, d'éthylène glycol, de propylène glycol et d'éther monobutylique d'éthylène glycol,

3. Procédé de formation d'un objet tridimensionnel selon la revendication 1, dans lequel la composition d'encre pour un support d'impression tridimensionnel comprend en outre un monomère comprenant au moins un groupe vinyle et un groupe acryle.

4. Procédé de formation d'un objet tridimensionnel selon la revendication 1, dans lequel R₂ et R₃ dans la formule chimique 1 représentent chacun indépendamment l'hydrogène, le méthyle, l'éthyle, le propyle, l'isopropyle, le n-butyle, le tert-butyle. 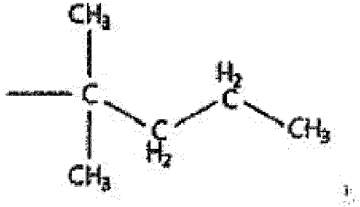 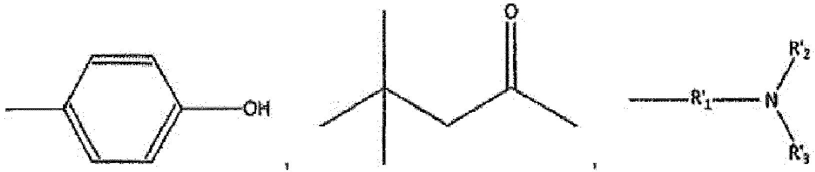 ou -R"₁----O---R"₂, dans laquelle formule R'₁ représente CH₂, CH₂CH₂, CH₂CH₂CH₂, CH(CH₂)CH₂, CH₂CH₂CH₂CH₂, CH₂C(CH₃)₂ ou C(CH₃)₂CH₂CH₂, R'₂ et R'₃ représentent chacun indépendamment l'hydrogène, CH₃, CH₂CH₃, CH₂CH₂CH₃, CH₂CH(CH₃)₂, CH₂C(CH₃)₃, CH(CH₃)₂, CH₂CH₂CH₂CH₃, C(CH₃)₂CH₂CH₃ ou -CH=CH₂, R"₁ représente CH₂, CH₂CH₂, CH₂CH₂CH₂, CH(CH₂)CH₂, CH₂CH₂CH₂CH₂, CH₂C(CH₃)₂ ou C(CH₃)₂CH₂CH₂, et R"₂ représente l'hydrogène, CH₃, CH₂CH₃, CH₂CH₂CH₃, CH₂CH(CH₃)₂, CH₂C(CH₃)₃, CH(CH₃)₂, (CH₂CH₂CH₂CH₃, C(CH₃)₂CH₂CH₃ ou -CH = CH₂.

5. Procédé de formation d'un objet tridimensionnel selon la revendication 1, dans lequel le composé de la formule chimique 1 est le composé de la formule chimique 1a : 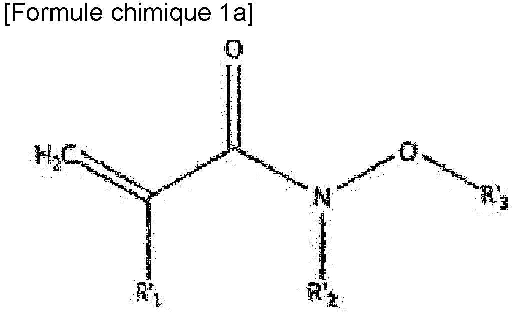 dans laquelle R'₁ représente l'hydrogène ou un groupe méthyle, et R'₂ et R'₃ représentent chacun indépendamment l'hydrogène, le méthyle, l'éthyle, le propyle, l'isopropyle, le n-butyle, le tert-butyle ou -CH = CH₂.

6. Procédé de fabrication d'un objet tridimensionnel selon la revendication 1, dans lequel R'₁ et R'₂ dans la formule chimique 2 représentent chacun indépendamment l'hydrogène, le méthyle, l'éthyle, le propyle, l'isopropyle, le n-butyle, le tert-butyle

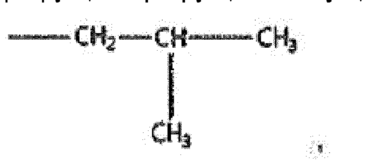

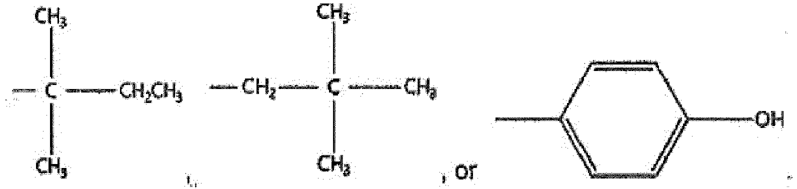

7. Procédé de fabrication d'un objet tridimensionnel selon la revendication 1, dans lequel le composé de la formule chimique 4 est un composé sélectionné parmi les composés suivants :

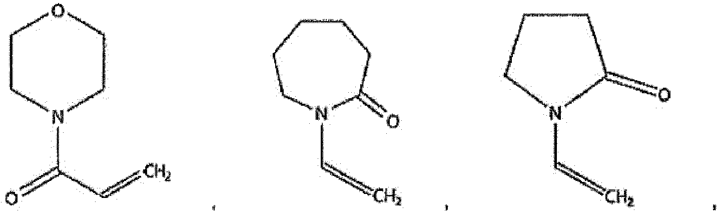

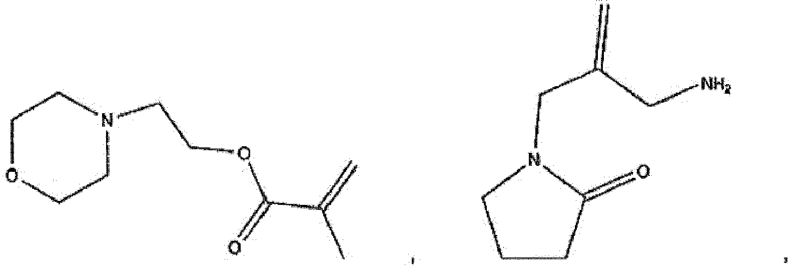

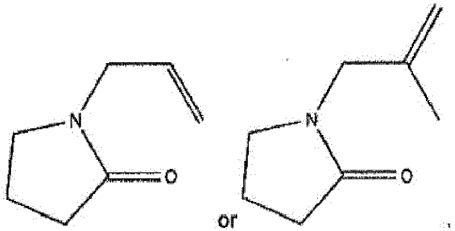

8. Procédé de fabrication d'un objet tridimensionnel selon la revendication 1, dans lequel le monomère contenant l'amine est contenu en une quantité de 10 à 99,9 % en poids par rapport au poids total de la composition.

9. Procédé de fabrication d'un objet tridimensionnel selon la revendication 1, dans lequel l'agent de durcissement est contenu en une quantité de 0,01 à 20 % en poids par rapport au poids total de la composition.
